# EUROPÄISCHE PATENTANMELDUNG

(11) **EP 2 033 680 A1**
(43) Veröffentlichungstag der Anmeldung: **11.03.2009**
(21) Anmeldenummer: 07017682.1
(22) Anmeldetag: 10.09.2007
(51) Int. Cl.: A61M 16/10, A61M 16/12, A61M 16/00

(54) **Xenon-Narkosebeatmungssystem**

(71) Anmelder: Hargasser, Stefan, Prof. Dr. med. Dr. med. habil., 81739 München (DE)
(72) Erfinder: Hargasser, Stefan, Prof. Dr. med. Dr. med. habil., 81739 München (DE)
(74) Vertreter: Vossius & Partner

(57) **Zusammenfassung**

Die Erfindung betrifft eine Vorrichtung zum Recyceln von Xenon in einem Narkosebeatmungssystem mit einem Rückhaltefilter (1), der dazu geeignet ist, Xenon zu adsorbieren und zu desorbieren, und einer an den Rückhaltefilter angeschlossenen ersten Leitung (2) zum Leiten eines von einem Patienten kommenden und Xenon enthaltenden ersten Atemgasgemisches in einer ersten Durchflussrichtung durch den Rückhaltefilter, wobei zumindest ein Teil des Xenons im Rückhaltefilter verbleibt. Ferner umfasst die Vorrichtung eine an den Rückhaltefilter angeschlossene zweite Leitung (3) zum Leiten eines zweiten Atemgasgemisches in einer zweiten Durchflussrichtung durch den Rückhaltefilter, wobei zumindest ein Teil des im Rückhaltefilter verbliebenen Xenons von dem zweiten Atemgasgemisch aufgenommen und mitgeführt wird und eine mit der ersten Leitung verbundene Zuleitung (4) zum Leiten eines Xenon enthaltenden Narkosemittels in die erste Leitung. Die Erfindung betrifft ferner eine Narkosebeatmungssystem mit einer obengenannten Vorrichtung sowie eine Methode zum Recyceln von Xenon in einem Narkosebeatmungssystem.

## Beschreibung

Die Erfindung betrifft eine Vorrichtung und ein Verfahren zum Recyceln von Xenon in einem Narkosebeatmungssystem sowie ein eine solche Vorrichtung enthaltendes Narkosebeatmungssystem.

Seit einigen Jahren wird der Einsatz von Xenon als Anästhetikum intensiv diskutiert. Der narkotische Effekt von Xenon ist rund 1,5 mal stärker als derjenige von Lachgas und wurde bereits in den 40-er Jahren nachgewiesen. Aufgrund der sehr geringen Blutlöslichkeit wird Xenon schneller abgeatmet als herkömmliche Anästhetika. Das hat den Vorteil, dass der Narkosezustand eines Patienten mit geringerer Verzögerung und somit effektiver reguliert werden kann. Außerdem wacht dadurch der Patient nach der Narkose rascher auf und verspürt dann deutlich weniger Nachwirkungen. Schädigende Nebenwirkungen sind bislang nicht bekannt, so dass Xenon bereits 2005 als Anästhetikum in Deutschland zugelassen wurde. Ein Xenon enthaltendes Anästhesiemittel ist beispielsweise in der WO 02/22116 offenbart.

Ein Nachteil der Verwendung von Xenon ist, dass Xenon üblicherweise in einem aufwendigen Verfahren mittels fraktionierter Verflüssigung aus Luft gewonnenen werden muss, was es zu einem kostenintensiven Rohstoff macht. Um die Wirtschaftlichkeit von Xenon-Narkosen zu verbessern, wurde deshalb über mögliche Wege der Rückgewinnung nachgedacht. So beschreibt beispielsweise die US 2005/0235831 A1 eine Vorrichtung und ein Verfahren zur Rückgewinnung von Xenon aus in Anästhesiegeräten eingesetzten Gasgemischen. Dabei wird eine externe Einrichtung zum Recyceln eingesetzt, die das Xenon aus dem bereits entsorgten Narkosegasgemisch mit Hilfe eines Zeolithen zurückgewinnt und unter Einsatz von Vakuum und Hitze reinigt bzw. konzentriert.

Das beschriebene Verfahren ist jedoch ausgesprochen aufwendig und die verwendete Vorrichtung entsprechend komplex. Das macht die Anwendung anfällig für Störungen und teuer.

Demnach ist es eine Aufgabe der vorliegenden Erfindung, ein verbessertes Verfahren sowie eine verbesserte und/oder einfachere Vorrichtung zum Recyceln von Xenon in Narkosebeatmungssystemen bereitzustellen, mit denen Patienten, wie Menschen und/oder alle Arten von Tieren einschließlich Säugern und Nichtsäugern, unter Narkose beatmet werden können. Diese Aufgabe wird mit den Merkmalen der Ansprüche gelöst.

Der Erfindung liegt der Gedanke zu Grunde, in dem Leitungsweg eines Narkosebeatmungssystems einen Rückhaltefilter bereitzustellen, der im Narkosegas enthaltenes Xenon aufnehmen, festhalten und wieder abgeben kann. Dadurch wird das Xenon intern recycelt, ohne dass zusätzliche externe Geräte oder Prozessschritte nötig wären. Dementsprechend umfasst die Vorrichtung zum Recyceln von Xenon gemäß einer bevorzugten Ausführungsform der Erfindung einen Rückhaltefilter, der dazu geeignet ist, Xenon zu adsorbieren und zu desorbieren, und eine an den Rückhaltefilter angeschlossene erste Leitung zum Leiten eines von einem Patienten kommenden und Xenon enthaltenden ersten Atemgasgemisches in einer ersten Durchflussrichtung durch den Rückhaltefilter, wobei zumindest ein Teil des Xenons im Rückhaltefilter verbleibt. Ferner umfasst die Vorrichtung zum Recyceln von Xenon eine an den Rückhaltefilter angeschlossene zweite Leitung zum Leiten eines zweiten Atemgasgemisches in einer zweiten Durchflussrichtung durch den Rückhaltefilter, wobei zumindest ein Teil des im Rückhaltefilter verbliebenen Xenons von dem zweiten Atemgasgemisch aufgenommen und mitgeführt wird, und eine mit der ersten Leitung verbundene Zuleitung zum Leiten eines Xenon enthaltenden Narkosemittels in die erste Leitung.

Bevorzugt weist die Vorrichtung ferner einen Probengasauslass auf, um einen Teil des ersten und/oder zweiten Atemgasgemisches aus der ersten Leitung einer Analyseeinrichtung zuzuführen. Letztere ermöglicht die Messung bzw. Überprüfung beispielsweise der Xenon-Konzentration im Atemgasgemisch, das dem Patienten zugeführt bzw. von diesem ausgeatmet wird, und gewährleistet somit einen adäquaten Narkosezustand.

Um das von der Analyseeinrichtung analysierte Gasgemisch und insbesondere das darin enthaltene Xenon zurückzugewinnen , wird dieses nach erfolgter Analyse wieder dem System zugeführt. Hierzu weist die Vorrichtung bevorzugt eine Verbindungsleitung zwischen der Analyseeinrichtung und der Zuleitung auf, um von der Analyseeinrichtung abgegebenes Atemgasgemisch wieder in die erste Leitung einzuleiten.

Ferner kann in die erste Leitung ein Filter zur Einstellung von Temperatur und/oder Feuchtigkeit des ersten und/oder zweiten Atemgasgemisches eingebracht werden. Ein zusätzlicher Kohlendioxidfilter bzw. -absorber kann ebenfalls vorgesehen sein. Optional sind der Filter zur Einstellung von Temperatur und/oder Feuchtigkeit und/oder der Kohlendioxidfilter zusammen mit dem Rückhaltefilter in einem Werkstück integriert.

Zur geeigneten Verabreichung des Narkosegasgemisches an den Patienten mündet die erste Leitung an ihrem patientenseitigem Ende vorzugsweise in einen endotrachealen Tubus, eine Larynxmaske oder eine andere Maske.

Der Rückhaltefilter umfasst im Wesentlichen ein organisches oder anorganisches Adsorptionsmittel, das dazu geeignet ist, Xenon zu adsorbieren und wieder zu desorbieren. Gleichzeitig soll der Rückhaltefilter für Sauerstoff, Kohlendioxid und Stickstoff im Wesentlichen durchlässig sein. Hierzu sind beispielsweise Aktivkohle, Kieselgel, Aluminiumoxid, Aluminiumsilikat, Zeolith oder ähnliche Materialen vorstellbar. Der im Rückhaltefilter adsorbierte Anteil des durchströmenden Xenons liegt vorzugsweise bei 50-100%, besonders bevorzugt bei 80-95%. Gegebenenfalls kann auf einen zu hohen Anteil verzichtet werden, um das Filtervolumen möglichst klein zu halten und dadurch den sogenannten Totraum, der sich ungünstig auf die Beatmung des Patienten auswirken kann, zu minimieren.

Da üblicherweise Xenon als Anästhetikum nicht alleine eingesetzt, sondern mit anderen Anästhetika gemischt verabreicht wird, können dem Narkosegas Sauerstoff, halogenierte Anästhetika, Desflurane, Sevofluran, Isofluran, Enfluran, Halothan oder eine Kombination mehrerer dieser Stoffe beigemischt sein.

Vorteilhafter Weise umfasst die Vorrichtung ferner eine Einrichtung zum Steuern der ersten und zweiten Durchflussrichtung. Dabei sind unterschiedliche Steuerungsmodi vorstellbar: Bevorzugt beginnt die Einleitung des Xenon enthaltenden Narkosegasgemisches am Beginn der Inspirationsphase, d.h. der Narkosegasfluss wird mit dem Atemzyklus des Patienten synchronisiert. Die applizierte Narkosegasmenge pro Zyklus entspricht dabei vorzugsweise 10-80% eines Inspirationsvolumens. Das führt zu einem Gasfluss von etwa 20 bis 60 *l*/min. Alternativ zu dieser Synchronsteuerung ist aber auch ein Modus vorgesehen, bei dem ein konstanter Xenon-Fluss bereitgestellt wird, was allerdings die Effizienz des Systems verringert.

Die vorliegende Erfindung betrifft außerdem ein Xenon-Narkosebeatmungssystem mit einer Vorrichtung zum Recyceln von Xenon wie oben beschrieben, einer Einrichtung zur Bereitstellung des zweiten Atemgasgemisches, einer Einrichtung zur Bereitstellung des Xenon enthaltenden Narkosemittels und einer Einrichtung zur Analyse eines entnommenen Atemgasgemisches.

Dabei weist die Analyseeinrichtung aus bereits genanntem Grund bevorzugt eine Einrichtung zur Bestimmung der Xenon-Konzentration in dem entnommenen Atemgasgemisch auf. Zusätzlich können aber auch Einrichtungen zur Messung von weiteren Parametern, z.B. der Konzentration anderer Narkosemittel, vorgesehen sein. Darüber hinaus ist insbesondere die Bestimmung der Sauerstoffkonzentration in dem entnommenen Atemgasgemisch notwendig.

Als Einrichtung zur Bereitstellung eines Atemgasgemisches kann ein herkömmliches Gerät verwendet werden. Dieses kann beispielsweise als Kreislaufsystem ausgelegt sein, das dazu geeignet ist, das gefilterte erste Atemgasgemisch aufzunehmen, über einen Kohlendioxidfilter bzw. -absorber zu leiten und wieder bereitzustellen. Vorgesehen sind aber auch Einwegsysteme, bei denen das ausgeatmete Atemgasgemisch nach Filterung durch den Rückhaltefilter abgeführt und entsorgt wird.

Die vorliegende Erfindung stellt zudem ein Verfahren zum Recyceln von Xenon aus einem Xenon enthaltenden ersten Atemgasgemisch in einem Narkosebeatmungssystem bereit. Das Verfahren umfasst die folgenden Schritte: Leiten des ersten Atemgasgemisches durch einen Rückhaltefilter in einer ersten Durchflussrichtung, wobei zumindest ein Teil des Xenons im Rückhaltefilter verbleibt, und Leiten eines Sauerstoff enthaltenden zweiten Atemgasgemisches durch den Rückhaltefilter in einer zweiten Durchflussrichtung, wobei zumindest ein Teil des im Rückhaltefilter verbliebenen Xenons in das zweite Atemgasgemisch abgegeben wird.

Beim Leiten des Atemgasgemisches durch den Rückhaltefilter treten dabei momentane Flüsse zwischen 20 und 60 *l*/min auf, bei einem typischen Atemminutenvolumen von 4 bis 14 *l*. Da die Atemminutenvolumina bei jungen Menschen oder Kleintieren deutlich kleiner sein können, beispielsweise zwischen 0,3 und 5 *l* für Kinder, ist es auch denkbar, spezielle kleinere Rückhaltefilter für diese Anwendungen bereitzustellen.

Ferner sind optional folgende weitere Schritte vorgesehen: Zuleiten des Xenon enthaltenden Narkosemittels in das aus dem Rückhaltefilter austretende zweite Atemgasgemisch, Entnehmen eines Probenvolumens aus dem zweiten Atemgasgemisch, Messen zumindest der Xenon-Konzentration des Probenvolumens und Rückführen des gemessenen Probenvolumens in das zweite Atemgasgemisch.

Außerdem kann die Temperatur und/oder Feuchtigkeit des ersten und/oder zweiten Atemgasgemisches eingestellt werden.

Soll zur Verminderung der Xenonkonzentration oder bei Beendigung der Narkose Xenon aus dem Patienten ausgewaschen werden, kann anstelle von Xenon Sauerstoff eingeleitet werden. Dabei beträgt das Volumen des eingeleiteten Sauerstoffs vorzugsweise mindestens 50% des Atemminutenvolumens.

Nachfolgend werden verschiedene Ausführungsformen der vorliegenden Erfindung anhand der Figuren beispielhaft beschrieben. Es zeigen:
Fig. 1: eine bevorzugte Ausführungsform einer erfindungsgemäßen Vorrichtung zum Recyceln von Xenon;
Fig. 2: einen Teil eines Narkosebeatmungssystems;
Fig. 3: eine Variante der bevorzugten Ausführungsform einer erfindungsgemäßen Vorrichtung zum Recyceln von Xenon; und
Fig. 4: eine weitere Ausführungsform einer erfindungsgemäßen Vorrichtung zum Recyceln von Xenon.

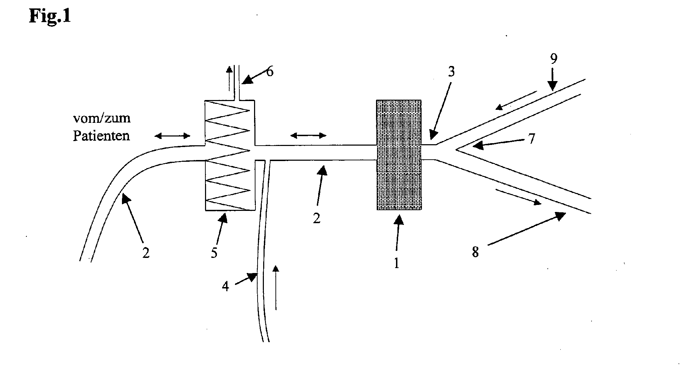

Figur 1 stellt schematisch eine bevorzugte Ausführungsform einer erfindungsgemäßen Vorrichtung zum Recyceln von Xenon dar. Diese umfasst einen Rückhaltefilter 1 und eine erste Leitung 2, die den Rückhaltefilter 1 mit einer Larynxmaske oder einem endotrachealen Tubus (nicht dargestellt) am patientenseitigen Ende der Leitung 2 zur Verabreichung eines Narkosegasgemisches an einen Patienten verbindet und ein vom Patienten kommendes und Xenon enthaltendes Atemgasgemisch in einer ersten Durchflussrichtung (hier: nach rechts) durch den Rückhaltefilter 1 leitet. Ferner ist an den Rückhaltefilter 1 eine zweite Leitung 3 angeschlossen, mittels derer ein zweites Atemgasgemisch in einer zweiten Durchflussrichtung (hier: nach links) durch den Rückhaltefilter 1 geleitet wird. Ein mindestens Xenon enthaltendes Narkosemittel wird über eine Zuleitung 4 in die erste Leitung 2 eingeleitet.

Der Patient atmet über die nicht dargestellte Maske ein Gasgemisch ein, das sich aus dem über die Leitung 3 zugeführten zweiten Atemgasgemisch und dem über die Zuleitung 4 zugeführten Narkosemittel zusammensetzt. Die Konzentrationsverhältnisse der Komponenten können über eine Steuerung geregelt werden. Das wieder ausgeatmete erste Atemgasgemisch wird über die Leitung 2 durch den Rückhaltefilter 1 geleitet, wobei ein Teil des im Gasgemisch enthaltenen Xenons im Rückhaltefilter 1 adsorbiert wird. Dieses adsorbierte Xenon kann im nächsten Atemzyklus zumindest teilweise wieder in das aus Leitung 3 stammende zweite Atemgasgemisch abgegeben werden. Überraschenderweise hat sich gezeigt, dass aufgrund sowohl der unterschiedlichen Strömungsgeschwindigkeiten als auch der jeweiligen differierenden Xenon-Konzentrationen der beiden Atemgasgemische der Rückhaltefilter für die beiden Durchflussrichtungen eine unterschiedliche Wirkung zeigt: Langsam strömendes Xenon-reiches Gas gibt bevorzugt Xenon an den Filter ab, wohingegen rascher strömendes Xenon-armes Gas dazu tendiert, Xenon vom Filter aufzunehmen. Dadurch wird das Xenon innerhalb eines Atemzyklusses recycelt.

Ferner weist die Vorrichtung eine Einrichtung 5 zum Austausch von Wärme und Feuchtigkeit auf. Diese erlaubt es beispielsweise, die im ausgeatmeten Atemgasgemisch enthaltene Feuchtigkeit teilweise aufzunehmen und wieder in das einzuatmende Atemgasgemisch abzugeben. An dieser Einrichtung ist ein Probenauslass 6 angebracht, um einen Teil des ersten und/oder zweiten Atemgasgemisches aus der ersten Leitung 2 einer Analyseeinrichtung (nicht dargestellt) zuzuführen. Letztere ermöglicht die Messung bzw. Überprüfung beispielsweise der Xenon-Konzentration im Atemgasgemisch, das dem Patienten zugeführt bzw. von diesem ausgeatmet wird, und gewährleistet somit einen adäquaten Narkosezustand. Die Anbringung des Auslasses 6 an der Einrichtung 5 bewirkt eine gute Durchmischung und somit die Entnahme einer repräsentativen Probe. Optional ist aber auch eine andere Anbringung denkbar.

Um das von der Analyseeinrichtung analysierte Gasgemisch und insbesondere das darin enthaltene Xenon zurückzuerhalten, wird dieses nach erfolgter Analyse wieder dem Kreislauf zugeführt. Hierzu weist die Vorrichtung bevorzugt eine Verbindungsleitung (nicht dargestellt) zwischen der Analyseeinrichtung und der Zuleitung 4 auf, um das von der Analyseeinrichtung abgegebene Atemgasgemisch wieder in die erste Leitung 2 einzuleiten.

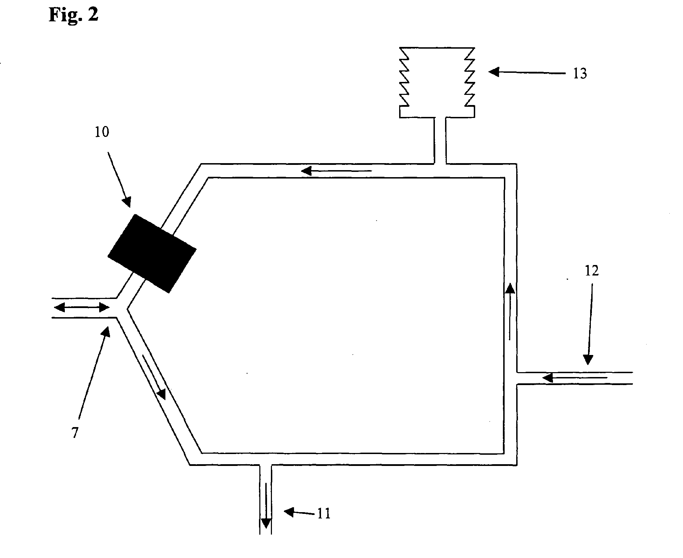

Die zweite Leitung 3 ist bevorzugt an ein Verzweigungs- bzw.Y-Stück 7 angeschlossen. Dies erlaubt beispielsweise die Ableitung und Entsorgung des verbrauchten Atemgases durch die Leitung 8 und Bereitstellung frischen Atemgases durch die Zuleitung 9. Für einen zyklischen Betrieb können die Leitungen 8 und 9 aber auch über eine entsprechende Steuerung miteinander verbunden sein. Dann empfiehlt sich das Einbringen eines Kohlendioxidfilters 10 in den Kreislauf, z.B. zwischen den Leitungen 8 und 9, beispielsweise wie in Figur 2 gezeigt.

Figur 2 stellt schematisch ein Narkosebeatmungssystem dar, an das eine Vorrichtung zum Recyceln von Xenon wie oben beschrieben über das Y-Stück 7 angeschlossen werden kann. Hier ist ein Kreislaufsystem gezeigt, in dem das ausgeatmete Atemgasgemisch zirkuliert und im Wesentlichen aufgefrischt wieder zum Einatmen bereitgestellt wird. Von dem Y-stück 7 strömt das Gemisch an einem Auslass 11 vorbei, an dem überschüssiges Gas abgelassen werden kann. Durch die Zuleitung 12 wird Frischgas, d.h. insbesondere Sauerstoff, aber gegebenenfalls auch Anästhetikum in den Kreislauf eingebracht. Der Beatmungsapparat 13 dient dazu, den momentanen Gasfluss von 20 bis 60 *l*/min bereitzustellen, der deutlich größer ist als der kontinuierliche Zufluss von Frischgas durch die Zuleitung 12, der sich typischerweise in der Größenordnung 1 *l*/min bewegt. Schließlich wird in einem Kohlendioxidfilter 10 das durch den Atmungsprozess angereicherte Kohlendioxid aus dem Atemgasgemisch herausgefiltert. Der entsprechende Volumenverlust wird durch besagten Zufluss durch die Zuleitung 12 kompensiert. Wird der Zufluss von Frischgas durch die Zuleitung 12 beispielsweise auf die Menge des Atemminutenvolumens erhöht, so erübrigt sich der Kohlendioxidfilter 10. Alternativ zu der Zuleitung von Frischgas durch die Zuleitung 12 ist es auch möglich, das Frischgas zusammen mit dem Narkosemittel durch die Zuleitung 4 (siehe Figur1) einzuleiten.

Natürlich wird in dem beschriebenen System die Flussrichtung in den einzelnen Leitungen durch entsprechende, nicht dargestellte Ventile geregelt. Ferner kann die Reihenfolge der Komponenten innerhalb des Kreislaufes variieren. So kann beispielsweise das Gemisch zuerst über den Kohlendioxidfilter 10 geleitet werden.

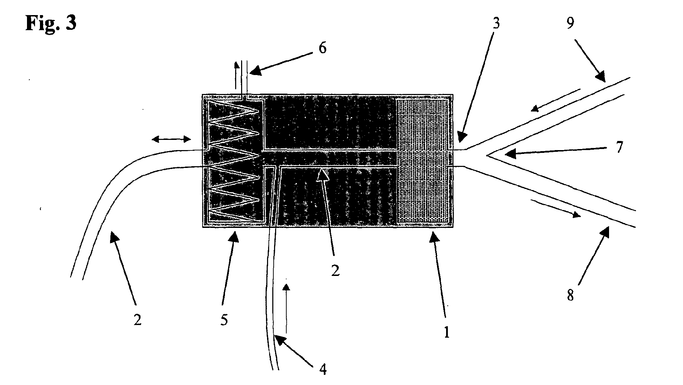

Figur 3 zeigt schematisch eine Variante der bevorzugten Ausführungsform gemäß Figur 1, bei der die Einrichtung 5 zum Austausch von Wärme und Feuchtigkeit sowie der Rückhaltefilter 1 in einem Stück integriert sind.

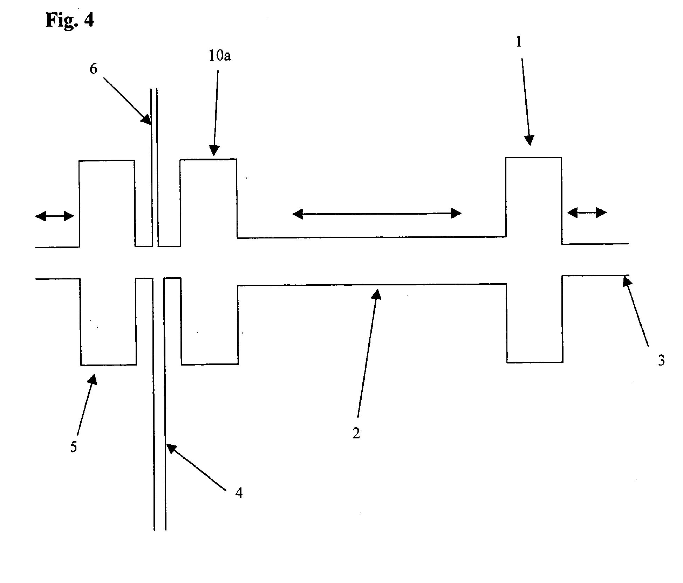

Figur 4 stellt schematisch eine weitere bevorzugte Ausführungsform einer erfindungsgemäßen Vorrichtung zum Recyceln von Xenon dar, die als Pendelsystem verwendet werden kann. Hierfür ist ein Kohlendioxidfilter bzw. -absorber 10a in der ersten Leitung 2 vorgesehen, der bezogen auf die Ausatemrichtung vor dem Rückhaltefilter 1 positioniert ist. Der Kohlendioxidfilter 10a kann aber auch hinter dem Rückhaltefilter 1 positioniert sein oder mit diesem zusammen einstückig ausgeführt sein. Im Gegensatz zu der Funktionsweise des Rückhaltefilters 1 wird vom Kohlendioxidfilter 10a CO₂ lediglich absorbiert, aber in Gegenstromrichtung nicht wieder an das Atemgasgemisch abgegeben. Als Materialen für einen Kohlendioxidfilter eignen sich beispielsweise Calciumhydroxid, Natriumhydroxid, Kaliumhydroxid, Bariumhydroxid o.ä., in denen CO₂ effektiv gebunden wird. Möglich ist aber auch die Verwendung eines wiederverwendbaren Kohlendioxidfilters, beispielsweise eines Zeolithen, der durch Wärmebehandlung wiederaufbereitet werden kann.

Durch die Filterung von CO₂ kann die Vorrichtung als Pendelsystem betrieben werden, d.h. der Patient atmet im Wesentlichen das zuvor in das System ausgeatmete Gemisch wieder ein: Das Atemgasgemisch "pendelt" in der Leitung 2 vor und zurück. Ohne den Kohlendioxidfilter würde sich bei einem solchen Betrieb das ausgeatmete CO₂ anreichern und in kürzester Zeit zu einer CO₂-Vergiftung führen. Der Xenon-Rückhaltefilter 1 verhindert dabei den diffusiven Verlust von Xenon in die Beatmungsmaschine. Geeigneter Weise sollte dabei das Volumen der ersten Leitung 2, des Kohlendioxidfilters 10a und der Einrichtung 5 zum Austausch von Wärme und Feuchtigkeit etwa dem Atemvolumen entsprechen und also vorzugsweise im Bereich von 0,2 bis 2,5 *l*, besonders bevorzugt im Bereich von 0,5 bis 1,2 *l* liegen. Hierfür ist die erste Leitung 2 im Bereich zwischen dem Rückhaltefilter 1 und dem Kohlendioxidfilter 10a vorzugsweise mit einem entsprechend vergrößerten Durchmesser und/oder einer geeigneten Länge versehen. Optional können auch der Rückhaltefilter 1 und der Kohlendioxidfilter 10a in einem entsprechend dimensionierten Gehäuse untergebracht sein, das dann als Leitung 2 fungiert, bzw. die beiden Filter können in die Leitung 2 integriert sein.

Um das gebundene CO₂ mit frischem Sauerstoff zu ersetzen, wird in dieser Ausführungsform Sauerstoff bevorzugt zusammen mit Xenon durch die Zuleitung 4 bereitgestellt. Der Pendelbetrieb verlangt nach einer patientennahen Zuleitung von Sauerstoff und gegebenenfalls weiterer Anästhetika wie Desfluran etc. Optional kann dafür auch eine weitere patientennahe Zuleitung vorgesehen sein. Der optionale Probengasauslass 6 kann, wie in Figur 4 dargestellt, direkt von der ersten Leitung 2 abzweigen oder aber gemäß Figur 1 an die Einrichtung 5 zum Austausch von Wärme und Feuchtigkeit angebracht sein. Vorzugsweise sind der Kohlendioxidfilter 10a, der Rückhaltefilter 1 sowie die Einrichtung 5 zum Austausch von Wärme und Feuchtigkeit in einem gemeinsamen Gehäuse mit entsprechenden Anschlüssen für die Zu- und Ableitungen untergebracht, ähnlich wie in Figur 3 gezeigt.

Ist der Kohlendioxidfilter 10a entsprechend Figur 4 relativ patientennah vorgesehen, können eventuell aus diesem austretende Atemkalkpartikel von der Einrichtung 5 zum Austausch von Wärme und Feuchtigkeit aufgehalten werden, so dass diese nicht in die Atemwege des Patienten gelangen.

Für den Pendelbetrieb kann die in Figur 4 dargestellte Ausführungsform an ein herkömmliches Narkosesystem, wie beispielsweise in Figur 2 dargestellt, angeschlossen werden. Allerdings wird dann der Einsatz des Kohlendioxidfilters 10 überflüssig, da ja bereits die Einrichtung 10a vor dem Rückhaltefilter 1 für entsprechende Filterung sorgt. Im Prinzip genügt jedoch eine einfache Pumpe inklusive Überdruckventil anstatt des in Figur 2 dargestellten Kreislaufes, die dazu geeignet ist, mit entsprechendem Saug- und Pumpverhalten, den notwendigen Pendelfluss zu erzeugen.

Die Vorrichtung der vorliegenden Erfindung erweist sich als vorteilhaft, da sie einfach und kostengünstig produzierbar ist und sich problemlos mit bereits vorhandenen Systemen kombinieren lässt. Die Vorrichtung ist leicht und kompakt und somit vielfältig und unkompliziert einsetzbar. Das Recycling geschieht intern, d.h. es sind keinerlei externe zusätzliche Geräte notwendig, um das Xenon zurückzugewinnen.

## Patentansprüche

1. Vorrichtung zum Recyceln von Xenon in einem Narkosebeatmungssystem, mit:
a) einem Rückhaltefilter (1), der dazu geeignet ist, Xenon zu adsorbieren und zu desorbieren;
b) einer an den Rückhaltefilter angeschlossenen ersten Leitung (2) zum Leiten eines von einem Patienten kommenden und Xenon enthaltenden ersten Atemgasgemisches in einer ersten Durchflussrichtung durch den Rückhaltefilter (1), wobei zumindest ein Teil des Xenons im Rückhaltefilter (1) verbleibt;
c) einer an den Rückhaltefilter (1) angeschlossenen zweiten Leitung (3) zum Leiten eines zweiten Atemgasgemisches in einer zweiten Durchflussrichtung in Richtung zum Patienten durch den Rückhaltefilter (1), wobei zumindest ein Teil des im Rückhaltefilter (1) verbliebenen Xenons von dem zweiten Atemgasgemisch aufgenommen und mitgeführt wird; und
d) einer mit der ersten Leitung (2) verbundenen Zuleitung (4) zum Leiten eines Xenon enthaltenden Narkosemittels in die erste Leitung (2).

2. Vorrichtung nach Anspruch 1, ferner mit einem Probengasauslass (6) zum Zuführen eines Teils des ersten und/oder zweiten Atemgasgemisches aus der ersten Leitung (2) zu einer Analyseeinrichtung.

3. Vorrichtung nach Anspruch 2, ferner mit einer Verbindungsleitung zwischen der Analyseeinrichtung und der Zuleitung (4), um von der Analyseeinrichtung abgegebenes Atemgasgemisch wieder in die erste Leitung (2) einzuleiten.

4. Vorrichtung nach einem der vorangegangenen Ansprüche, wobei die erste Leitung (2) einen Filter (5) zur Einstellung von Temperatur und/oder Feuchtigkeit des ersten und/oder zweiten Atemgasgemisches aufweist.

5. Vorrichtung nach Anspruch 4, wobei der Filter (5) zur Einstellung von Temperatur und/oder Feuchtigkeit und der Rückhaltefilter (1) in einem Werkstück integriert bzw. in ein gemeinsames Gehäuse eingebaut sind.

6. Vorrichtung nach einem der vorangegangenen Ansprüche, wobei die erste Leitung (2) an ihrem patientenseitigen Ende einen endotrachealen Tubus, eine Larynxmaske oder eine andere Maske aufweist.

7. Vorrichtung nach einem der vorangegangenen Ansprüche, wobei der Rückhaltefilter (1) ein organisches oder anorganisches Adsorptionsmittel aufweist.

8. Vorrichtung nach einem der vorangegangenen Ansprüche, wobei der Rückhaltefilter (1) mindestens ein Material aus der folgenden Gruppe aufweist: Aktivkohle, Kieselgel, Aluminiumoxid, Aluminiumsilikat, Zeolith.

9. Vorrichtung nach einem der vorangegangenen Ansprüche, wobei das Narkosemittel zusätzlich zu Xenon einen oder mehrere Stoffe der folgenden Gruppe aufweisen kann: Sauerstoff, halogenierte Anästhetika, Desflurane, Sevofluran, Isofluran, Enfluran, Halothan.

10. Vorrichtung nach einem der vorangegangenen Ansprüche, ferner **gekennzeichnet durch** eine Einrichtung zum Steuern der ersten und zweiten Durchflussrichtung.

11. Vorrichtung nach einem der vorangegangenen Ansprüche, wobei die erste Leitung (2) ferner einen Kohlendioxidfilter (10a) aufweist.

12. Vorrichtung nach Anspruch 11, wobei der Kohlendioxidfilter (10a) bezogen auf die erste Durchflussrichtung vor oder hinter dem Xenon-Rückhaltefilter (1) angebracht ist.

13. Vorrichtung nach Anspruch 11 oder 12, wobei der Kohlendioxidfilter (10a) und der Rückhaltefilter (1) in einem Werkstück integriert bzw. in ein gemeinsames Gehäuse eingebaut sind.

14. Vorrichtung nach einem der Ansprüche 11 bis 13, wobei der Kohlendioxidfilter (10a) Atemkalk enthält, der mindestens ein Material aus der folgenden Gruppe aufweist: Calciumhydroxid, Natriumhydroxid, Kaliumhydroxid, Bariumhydroxid.

15. Vorrichtung nach einem der Ansprüche 11 bis 14, wobei der Kohlendioxidfilter (10a) ein wiederverwendbarer Filter ist, der mittels Wärmebehandlung aufbereitet werden kann.

16. Vorrichtung nach einem der Ansprüche 11 bis 15, wobei die erste Leitung (2) zwischen dem Kohlendioxidfilter (10a) und dem Rückhaltefilter (1) so ausgelegt ist, dass das Gasvolumen innerhalb der ersten Leitung (2) einschließlich des Kohlendioxidfilters (10a) zwischen 0,2 und 2,5 *l*, vorzugsweise zwischen 0,5 und 1,2 *l* beträgt.

17. Xenon-Narkosebeatmungssystem, mit:
a) einer Vorrichtung zum Recyceln von Xenon nach einem der Ansprüche 1 bis 16;
b) einer Einrichtung zur Bereitstellung des zweiten Atemgasgemisches;
c) einer Einrichtung zur Bereitstellung des Xenon enthaltenden Narkosemittels; und
d) einer Einrichtung zur Analyse eines entnommenen Atemgasgemisches.

18. Narkosesystem nach Anspruch 17, wobei die Analyseeinrichtung eine Einrichtung zur Bestimmung der Xenon-Konzentration in dem entnommenen Atemgasgemisch aufweist.

19. Narkosesystem nach Anspruch 17 oder 18, wobei die Einrichtung zur Bereitstellung des zweiten Atemgasgemisches als Kreislaufsystem ausgelegt ist, das dazu geeignet ist, das gefilterte erste Atemgasgemisch aufzunehmen, über einen Kohlendioxidfilter (10) zu leiten und als zweites Atemgasgemisch wieder bereitzustellen.

20. Narkosesystem nach einem der Ansprüche 17 bis 19, ferner mit einer Ableitung zum Abführen und/oder Entsorgen des gefilterten ersten Atemgasgemisches.

21. Narkosesystem nach einem der Ansprüche 17 bis 20, ferner mit einer Vorrichtung zur Bereitstellung eines Gasflusses zwischen 20 und 60 *l*/min in die zweite Leitung (3).

22. Verfahren zum Recyceln von Xenon aus einem Xenon enthaltenden ersten Atemgasgemisch in einem Narkosebeatmungssystem, mit den Schritten:
a) Leiten des ersten Atemgasgemisches durch einen Rückhaltefilter in einer ersten Durchflussrichtung, wobei zumindest ein Teil des Xenons im Rückhaltefilter verbleibt; und
b) Leiten eines Sauerstoff enthaltenden zweiten Atemgasgemisches durch den Rückhaltefilter in einer zweiten Durchflussrichtung, wobei zumindest ein Teil des im Rückhaltefilter verbliebenen Xenons in das zweite Atemgasgemisch abgegeben wird.

23. Verfahren nach Anspruch 22, ferner mit den Schritten:
a) Zuleiten des Xenon enthaltenden Narkosemittels in das aus dem Rückhaltefilter austretende zweite Atemgasgemisch;
b) Entnehmen eines Probenvolumens aus dem zweiten Atemgasgemisch;
c) Messen zumindest der Xenon-Konzentration des Probenvolumens; und
d) Rückführen des gemessenen Probenvolumens in das zweite Atemgasgemisch.

24. Verfahren nach Anspruch 22 oder 23, ferner mit dem Schritt: Einstellen von Temperatur und/oder Feuchtigkeit des ersten und/oder zweiten Atemgasgemisches.
